# EUROPÄISCHE PATENTSCHRIFT

(11) **EP 1 502 875 B1**
(45) Veröffentlichungstag und Bekanntmachung des Hinweises auf die Patenterteilung: **01.03.2006**
(21) Anmeldenummer: 04090276.9
(22) Anmeldetag: 14.07.2004
(51) Int. Cl.: B65D 81/32, A45D 40/00

(54) **Packungseinheit mit kosmetisch wirksamen Bestandteilen**
Packing unit with cosmetic active components
Système d'emballage avec composants cosmétiques

(30) Priorität: 23.07.2003 DE 10334139; 27.11.2003 DE 10356692
(43) Veröffentlichungstag der Anmeldung: 02.02.2005
(73) Patentinhaber: Coty B.V., 2031 CC Haarlem (NL)
(72) Erfinder: Bleuez, Lois, 06700 Sait Laurent du Var (FR); Porcu, Maryse, 06240 Beausoleil (FR)
(74) Vertreter: Walter, Wolf-Jürgen

(56) Entgegenhaltungen:
- WO-A-00/66455
- WO-A-79/00483
- DE-A- 19 809 942
- US-A- 2 899 318
- US-A1- 2002 046 566

## Beschreibung

Die Erfindung betrifft eine kosmetische Packungseinheit, bestehend aus zwei ineinander angeordneten, verschlossenen Behältern, deren Inhaltsstoffe voneinander getrennt sind.

Aus der US 6269654 Bl ist eine Folie mit einzelnen Abschnitten bekannt, auf denen ein wasserabsorbierendes, zellulosefreies Material angeordnet ist, das von einer weiteren wasserdurchlässigen Folie in diesen Abschnitten abgedeckt und versiegelt ist. Nach Kontakt mit Wasser füllen sich die einzelnen Abschnitte und ergeben jeweils eine kissenartige Struktur. Ein solche Folie mit einer Vielzahl von "Kissen" kann erwärmt oder gekühlt werden und dient zur vorübergehenden Kühlung oder Erwärmung darin eingeschlagener Produkte.

Bei anderen Anwendungsformen werden kosmetische Wirkstoffe in zwei Behälter verpackt und dann im Anwendungsfall geöffnet und miteinander vermischt, wie in der WO O1/04548, wobei ebenfalls Erwärmungseffekte durch eine exotherme Reaktion von bestimmten Inhaltsstoffen wie Calciumoxid mit Wasser angestrebt werden. Es sind auch Lösungen wie in der DE 19809942 A1 bekannt, bei denen räumlich getrennte Produkte erst im Anwendungsfall miteinander vermischt werden.

Die US 6508604 B1 beschreibt einen verschlossenen wasserundurchlässigen Behälter, in dem ein wasserdurchlässiger Behälter mit kosmetischen Substanzen enthalten ist.

Aufgabe der vorliegenden Erfindung ist es, kosmetische Substanzen, die in wäßriger oder trockener Zubereitung Stabilitäts-oder Kompatibiltätsprobleme hervorrufen können, in einer stabilen und leicht anwendbaren Form zu präsentieren und in wäßriger Phase auf der Haut anwendbar zu machen.

Eine weitere Aufgabe besteht in der Bereitstellung eines Erzeugnisses mit Frischeeffekt.

Erfindungsgemäß besteht die kosmetische Packungseinheit aus zwei ineinander angeordneten Behältern, wobei der erste Behälter aus einem wasserdichten Material besteht und der zweite Behälter aus einem wasserdurchlässigen Material besteht und in dem ersten Behälter angeordnet ist, und beide Behälter verschlossen sind,
dadurch gekennzeichnet, daß der erste Behälter standfähig ist oder zu einer standfähigen Form entfaltbar ist und Mittel zu seiner Öffnung aufweist, und der zweite Behälter in seinem Inneren einen geträgerten oder ungeträgerten, teilchenförmigen, kosmetisch wirksamen Stoff oder ein Stoffgemisch enthält und einen zweiten teilchenförmigen Stoff enthält, der bei Kontakt mit Wasser ein wenigstens um das Vierfache vergrößertes Volumen hat.

Besonders vorteilhaft hat der zweite teilchenförmige Stoff bei Kontakt mit Wasser ein wenigstens um das 10-fache vergrößertes Volumen.

Der zweite teilchenförmige Stoff kann eine Absorptionsfähigkeit für Wasser im Bereich des 20- bis 50-fachen seines Trockengewichtes haben.

Es wird weiterhin bevorzugt, daß die Absorptionsfähigkeit für Wasser einer vorgegebenen Menge des zweiten teilchenförmigen Stoffes in einem Zeitraum von 5 bis 150 Sekunden liegt.

Ein besonderer Vorteil der Erfindung besteht darin, daß eine wäßrige Zubereitung, bei der normalerweise bei längerer Lagerungszeit Stabilitätsprobleme durch z.B. Hydrolyse auftreten würde, in einer Trockenform vorliegt, z.B. auf einem pulverförmigen Träger aufgebracht ist. Sie wird in diese kosmetische Packungseinheit trocken eingebracht und erst unmittelbar vor der Anwendung mit Wasser in Kontakt gebracht und dann in einer praktischen Anwendungsform auf die Haut feucht aufgetragen.

Bei bestimmten Anwendungsformen, wie Parfüms, ist die Vermeidung von Estern möglich, und ebenso können Stabilitätsprobleme von Vitamin C oder Dihydroxyaceton (DHA) in Gegenwart von Wasser mit Hilfe der vorliegenden Erfindung ausgeschlossen werden.

Ein weiterer interessanter Aspekt bezüglich der erfindungsgemäßen Packungseinheit ist die Weichheit beim Reiben oder bei der einfachen Anwendung auf der Körper- oder Gesichtshaut oder auf dem Haar, was zu einer sehr angenehmen kosmetischen Sinneswahrnehmung führt.

Die erfindungsgemäße Packungseinheit eignet sich besonders zum Aufbringen von Deodorants, Parfüms, Parfümhilfsstoffen, Sonnenschutzmitteln, Hautschutz allgemein, Detergentformulierungen für Gesicht, Körper und Haar, Make-up, Make-up Entferner, Rasierhilfen, anderer Detergents und Reinigungsmittel usw. Sie kann auch als kosmetische Maske mit entsprechender Größe und entsprechendem Zuschnitt mit Öffnungen für Augen, Nase und Mund eingesetzt werden.

Die erfindungsgemäße Packungseinheit kann einfach auch nur als Frische-Effekt infolge der Einwirkung von Wasser und/oder spezieller zugesetzter kosmetischer Bestandteile auf die Körper-oder Gesichtshaut oder auf das Haar eingesetzt werden. Der Frische-Effekt kann durch Mentholderivate erhöht werden.

Ein besonderer Vorteil besteht darin, daß die erfindungsgemäße Packungseinheit nach dem Öffnen und Wasserkontakt in Form eines handlichen, weichen Vlieses vorliegt, und dadurch die gezielte Anwendung bestimmter kosmetischer Stoffe auf einzelne ausgewählte Hautpartien genau abgrenzbar ermöglicht wird.

Eine weitere innovative Anwendungsmöglichkeit besteht in der spezifischen Qualität des Faservlieses (non-woven), das zusätzlich zu anderen beschriebenen Bestandteilen kleine Kügelchen oder Granulatteilchen eingebettet enthalten kann, was einen reinigenden Abriebeffekt der Packungseinheit bewirkt. Das Faservlies kann mit Bestandteilen formuliert werden, die einen Versiegelungseffekt herbeiführen können, wie zum Beispiel Polyamide, Polyoxymethylen, Ethylen-Polyterephthalat, Butylen-Polyterephthalat, Polyetetrafluoroethylen, Polypropylen, HD-oder LD-Polyethylen und Polyvinylclorid. Der unter Wärmewirkung durchzuführende Versiegelungsprozeß kann durch Widerstandssiegeln, Ultraschall oder mittels magnetischer. Systeme bewirkt werden.

In einer besonders bevorzugten Ausführungsform besteht der erste Behälter aus einer Aluminiumfolie, die mit PE und/oder PET beschichtet ist. Am wichtigsten ist dabei nicht das Material, sondern die angepaßte Größe für die Packung zwecks leichter Handhabung, deren Fähigkeit für einen festen Stand, die gute Durchlässigkeit für und Kompatibilität mit kosmetische(n) Bestandteile(n) und ein leicht zu öffnendes System.

Die Herstellung der Packungseinheit erfolgt in der Weise, daß beispielsweise ein polypropylenhaltiges Faservlies mit entsprechender gewünschter Zuschnittgröße, z.B. 75 x 85 mm, in doppelter Lage durch Heißsiegelung zu einem auf drei Seiten verschlossenen Beutel verarbeitet wird. Dann erfolgt die Füllung des flachen Beutels mit dem entsprechenden Trockengemisch aus kosmetischem Wirkstoff und wasserabsorbierendem Stoff. Anschließend wird die vierte Seite des Beutels verschlossen, und der flexible gefüllte Beutel (zweiter Behälter) in den größeren (ersten) Behälter gegeben, der aus wasserdichtem Material besteht. Dieser äußere (erste). Behälter wird dann verschlossen, und er ist mit Mitteln zur Öffnung des ersten Behälters versehen.

Der größere (erste) Behälter muß eine standfähige Form haben oder zu einer standfesten Form entfaltbar sein, z.B. mit einer Bodenfalte versehen sein im Falle einer Folie, die aus Aluminium mit Beschichtungen aus Polyethylen (PE) oder PET (Polyethylenterephthalat) bestehen kann. Diese Standfähigkeit ist erforderlich, damit der äußere Behälter im Anwendungsfalle nach dem Öffnen mit einer vorgegebenen Menge Wasser gefüllt werden kann, um die Wasserabsorption durch den absorptionsfähigen Stoff in dem inneren Behälter zu ermöglichen.

Die Menge an absorptionsfähigem Stoff in dem inneren Behälter ist so eingestellt, daß entsprechend dem ausgewählten Absorptionsmittel mit der vorgegebenen Menge Wasser eine Absorption innerhalb kurzer Zeit, z.B. in 10 bis 200 Sekunden, vorzugsweise 30 bis 150 Sekunden, erfolgen kann. Durch den Wasserkontakt und der damit verbundenen Volumenvergrößerung erhält der innere Behälter eine Kissenform, kann in dieser feuchten Form leicht aus dem äußeren Behälter entnommen werden und mit der Haut durch Auflegen oder vorzugsweise leichtes Reiben in Kontakt gebracht werden.

Als absorptionsfähiger Stoff eignet sich besonders Natriumpolyacrylat oder modifizierte Carboxymethylcellulose. Beides sind Handelsprodukte und sind in Mengen von 1-90 Gew-% einsetzbar, vorteilhaft 10 bis 50 Gew-%. Bei Mengen von unter 1% sind die Wartezeiten für das Erreichen einer vollständigen Absorption meist zu lang, so daß solche niedrigen Konzentrationen nicht bevorzugt sind. Bei Mengen >50% wird die Absorption bereits nach wenigen Sekunden erreicht, und es ist kein signifikanter Vorteil mehr erreichbar - oder die absolute Menge des absorptionsfähigen Materials kann deutlich verringert werden.

Speziell bevorzugte Produkte sind Flocare® DP/GB300 und G800, die wasserunlösliche Pulver mit Teilchengrößen von 300 µm bzw. bis zu 800 µm darstellen. Die Absorption beträgt 170 g/g bzw. ≥ 400 g/g. Weiterhin bevorzugt ist Aquasorb® mit dem INCI-Namen Cellulose, Carboxymethyl ether, Sodium Salt und mit den Qualitäten A380 und A500. Tests haben ergeben, daß diese Stoffe in der Lage sind, Wasser zu absorbieren, das NaCl (0.9 % w/w) enthält.

Eine Verbesserung kann bei der Wasserabsorption weiterhin dadurch erreicht werden, daß neben dem wasserabsorptionsfähigen Stoff ein spezielles Faservlies mit hohen Wasserabsorptions-Eigenschaften für den inneren Behälter verwendet wird, wie z.B. das Faservlies A4459 von Ahlström (Helsinki, Finnland). Die gute Wasserabsorption und sichere Wasserdurchlässigkeit führt zu einem ausgezeichneten Kontakt des Inhalts im Faservliesbehälter mit Wasser.

Die in dem inneren Behälter enthaltenen kosmetischen Wirkstoffe, wie Parfüm, Panthenol, DHA, Vitamin F, AHA oder ein anderes Feuchthaltemittel, kommen dadurch in Kontakt mit der Haut und können ihre vorgesehene Wirkung entfalten. Die Feuchtigkeit des "Kissens" führt zusätzlich zu einem Erfrischungseffekt auf den ausgewählten Hautflächen.

Für den Fall, daß die kosmetischen Wirkstoffe flüssig oder halbflüssig sind, wie z.B. Parfüm, D-Panthenol, Vitamin C-Ester, Retinol und Retinolderivate, Tocopherol und Tocopherolderivate, Feuchthaltemittel, Erweichungsmittel, α-Bisabolol, Antioxidantien, Radikalfänger, bestimmte Pflanzenextrakte usw" werden diese auf einen pulverförmigen Träger aufgebracht, z.B. auf ein modifiziertes Silica von Degussa (Deutschland).

Dies kann sowohl mit Flüssigkeiten auf wäßriger Basis, auf öliger Basis oder auf Basis von organischen Lösungsmitteln erfolgen. Derartige Träger sind z.B. Nylon-12, Diatomeen- erde, Kaolin, Spherolin K, PMMA wie Methyl Methacrylate Crosspolymer, Glimmer, Talkum, Bornitrid (Keramik), Polytrap (Laurylmethacrylate/Glycol Dimethacrylate Crosspolymer), CMC modifiziert oder Absorptions-ähnliche mikrokristalline Zellulose, bentonit oder verschiedene Tone, Zeolithe, Absorbants POLYPORE E200 & L200, Patapos-Stärke, Tapioca-Stärke, Carbonate Acrylate Copolymer, Siliciumdioxid (silica), modifiziertes Siliciumdioxid, Dextrine und Polydextrine.

Modifiziertes Silica oder Nylon-12 sind bevorzugt. Träger wie Siliciumdioxid können das 1,5-fache ihres Eigengewichtes an Zusatzstoffen aufnehmen.

Falls die kosmetischen Wirkstoffe teilchenförmig sind, wie z.B. DHA, Vitamin C, bestimmte sprühgetrocknete Pflanzenextrakte usw., können sie ohne Träger in den inneren Behälter eingebracht werden, separat oder im Gemisch mit dem absorptionsfähigen Material.

Der Anteil der kosmetisch annehmbaren Substanzen, die bei Umgebungstemperatur fest oder flüssig sind, liegt im Bereich von 0,1 bis 95 Gew-%, bezogen auf das Gesamtgemisch der Behälterinhalte.

Bei Kontakt von Wasser und dem absorptionsfähigen Material findet im wesentlichen keine Temperaturveränderung statt, d.h. das zugegebene Wasser behält seine ursprüngliche Temperatur. Die Auswahl der kosmetischen Bestandteile erfolgt in der Weise, daß auch keine Reaktion dieser Stoffe im wäßrigen Medium mit anderen vorhandenen Stoffen erfolgt. Der einzige Weg für Temperaturerhöhungen als exotherme Reaktion könnte im Falle eines Säureeinsatzes, wie einer α-Hydroxysäure wie Glycolsäure, in Abhängigkeit von deren Konzentration auftreten oder bei bestimmten Haarentfernungsmittel, die Ca(OH)₂n NaOH oder KOH enthalten. Endotherme Reaktionen könnten andererseits bei der Formulierung von beispielsweise harnstoffhaltigen Feuchthalteprodukten auftreten.

Die kosmetische Zusammensetzung kann vorteilhaft auch Antioxidationsmittel und Radikalfänger enthalten. Zu derartigen Substanzen gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Äpfelsäure; Stilbene und deren Derivate.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na-oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Oberflächenaktive Mittel mit Aminosäuren sind zum Beispiel Protelan AGL95 oder Amisoft LS-22 sowie nichtionische oberflächenaktive Mittel wie Cremophor CO 40 oder 60, Emulgin L, Tween 20, Brij 30, 96 oder 98, Mergital LM3 oder Arlypon F, die bevorzugt sind. Für Reinigungsapplikationen werden vorzugsweise anionische und amphotere oberflächenaktive Mittel eingesetzt. Ein Gemisch von anionischen mit amphoteren oberflächenaktiven Mitteln ist ebenfalls bevorzugt, wie Disodium Lauroamphodicetate und Sodium Laureth Sulfate (Rewoteric® AM G30) oder Plantaren® XLS, Plantaren® TLS, Standapol® AP Gemisch.

Ein bevorzugter Bereich für den Einsatz dieser bevorzugten Mittel sind 1 bis 10 Gew-%. Zur Erreichung von Konditionierungseffekten werden übliche Hilfsstoffe verwendet, vorzugsweise kationische Bestandteile, wie Polyquaternium 7, 10, 11, 16, 39 oder 44, und Siliconpolymere mit unterschiedlichem Molekulargewicht, vorzugsweise mit hohem Molekulargewicht wie DC HMW 2220 oder eine Assoziation von Silicon und quaternisierten Polymeren.

Eine Vielzahl von anionischen oberflächenaktives Mittel ist potentiell hier einsetzbar. Zu nicht einschränkenden Beispielen von anionischen schäumenden oberflächenaktives Mitteln gehören solche, die aus der Gruppe ausgewählt sind, bestehend aus Alkyl-und Alkylethersulfaten, sulfatierten Monoglyceriden, sulfonierten Olefinen, Alkylarylsulfonaten, primären oder sekundären Alkansulfonaten, Alkylsulfosuccinaten, Acyltauraten, Acylisothionaten, Alkylglycerylethersulfonaten, Sulfonatmethylestern, sulfonierten Fettsäuren, Alkylphosphaten, Acylglutamaten, Acylsarcosinaten, Alkylsulfoacetaten, acylierten Peptiden, Alkylethercarboxylaten, Acyllactylate anionischer fluorhaltiger oberflächenaktiver Mittel und Gemische davon. Gemische von anionischen oberflächenaktiven Mitteln können wirksam in der vorliegenden Erfindung eingesetzt werden.

Zu Beispielen von amphoteren oberflächenaktiven Mitteln, die in der vorliegenden Erfindung verwendet werden können, gehören wenigstens solche mit einer Säuregruppe. Dies kann eine Carboxylgruppe oder eine Sulfonsäuregruppe sein. Eingeschlossen sind quaternärer Stickstoff, und daher auch quaternäre Aminosäuren. Sie sollten generell eine Alkyl- oder Alkenylgruppe mit 7 bis 18 Kohlenstoffatomen enthalten. Zu geeigneten amphoteren Detergentien gehören einfache Betaine und Amidobetaine, die ein Gemisch von C12- und C14-Alkylgruppen darstellen, die von der Kokosnuß abgeleitet sind, so daß wenigstens die Hälfte, vorzugsweise drei Viertel der R1-Kohlenwasserstoffkette 10 bis 14 Kohlenstoffatom hat. Die anderen beiden R2- und R3-Kohlenwasserstoffketten sind vorzugsweise Methyl. Eine weitere Möglichkeit besteht darin, daß das amphotere Detergenz ein Sulfobetain ist. Amphoacetate und Diamphoacetate können auch als mögliche zwitterionische und/oder amphotere Verbindungen auftreten, die eingesetzt werden können. Ein amphoteres oberflächenaktives Mittel sollte allgemeinen mit etwa 0,1 to 20%, vorzugsweise 5 bis 18 Gew-% enthalten sein, bezogen auf die Zusammensetzung.

Zu geeigneten nichtionischen oberflächenaktiven Mitteln gehören, sind allerdings nicht darauf beschränkt, Kokosnuß-acylmono- oder -diethanolamide, Alkylpolysaccharide, Lactobionamide, Ethylenglycolester, Glycerinmonoether, Polyhydroxyamide (Glucamide), primäre und sekundäre Alkoholethoxylate, insbesondere die C₈₋₂₀ aliphatischen Alkohole die durchschnittliche mit 1 bis 20 Molen Ethylenoxide pro Mol Alkohol ethoxyliert sind. Es können auch Gemische der zuvor genannten oberflächenaktiven Mittel eingesetzt werden.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Bäumwollsamenöl, Olivenöl, mineralische Öle, Butylmyristat, Palmitinsäure usw.

Geeignete Feuchthaltemittel sind beispielsweise Glycerin, Butylenglycol, Propylenglycol und Gemische davon.

Vorteilhaft können auch DHA, seine Vorläufer, Melanin oder andere kosmetische Wirkstoffe mit hautfärbender oder auch mit hautaufhellender Wirksamkeit eingesetzt werden.

Gegenstand der Erfindung ist auch die Verwendung der Packungseinheit zur Anwendung auf der Haut oder dem Haar zur Erzeugung eines Frische-Effektes.

Die Erfindung soll nachstehend durch Beispiele näher erläutert werden. Alle Angaben erfolgen in Gewichtsprozent, sofern nichts anderes angegeben ist.

### Beispiel 1

Aus einem PP-Faservlies der Qualität A4459 (Ahlström), das eine vollständige Durchlässigkeit für Wasser und eine Absorptionskapazität von 550% hat und synergistisch mit dem wasserabsorbierenden teilchenförmigen Material zusammenwirkt, wird ein auf drei Seiten verschlossener Beutel hergestellt. Der Beutel wird gefüllt mit 1,75 g eines teilchenförmigen Materials mit einer Zusammensetzung gemäß den folgenden Beispielen. Der Beutel wird durch Heißsiegeln verschlossen und hat eine Größe von 85 x 75 mm.

Der Beutel wird als innerer Behälter in einen größeren Beutel von etwa 95 x 140 mm gegeben, dessen Boden auffaltbar ist und diesem äußeren Behälter dadurch Standfähigkeit verleiht. Der größere Beutel (äußerer Behälter) besteht aus PET/Aluminium/-PE-Folie und wir ebenfalls verschlossen, z.B. durch einen Laserstrahl. Dieser äußere Behälter weist an seinem oberen Ende einen Öffnungsmechanismus auf, z.B. ein Aufreißband (ZIP LOG), das im Anwendungsfalle betätigt wird.

Nach dem Öffnen des äußeren Behälters wird eine vorbestimmte Menge Wasser zugegeben, z.B. etwa 60 ml, und nach einer Wartezeit von ca. 5-150 Sekunden ist die Absorption des Wassers abgeschlossen, und der innere Behälter, der inzwischen eine Kissenform angenommen hat, kann zu Anwendung entnommen werden.

### Beispiel 2 Erfrischungsmittel mit Vitamin C

Der innere Behälter wird mit zwei Phasen teilchenförmiger Materialien gefüllt. Es wird wie im Beispiel 1 gearbeitet.

| **Phase A** | |
|---|---|
| Ascorbinsäure | 10 |

| **Phase B** | |
|---|---|
| Sodium Polyacrylate (Flocare DP/GB 300 von SNF) | 90 |

### Beispiel 3 Bräuner

Der innere Behälter wird mit zwei Phasen teilchenförmiger Materialien gefüllt. Es wird wie im Beispiel 1 gearbeitet.

| **Phase A** | |
|---|---|
| Dihydroxy Acetone | 90 |

| **Phase B** | |
|---|---|
| Sodium Polyacrylate (Flocare DP/GB 300 von SNF) | 10 |

### Beispiel 4 Parfüm

Der innere Behälter wird mit zwei Phasen teilchenförmiger Materialien gefüllt. Es wird wie im Beispiel 1 gearbeitet.

| **Phase A** | |
|---|---|
| Parfüm (verkapselt in β-Cyclodextrin) | 60 |

| **Phase B** | |
|---|---|
| Sodium Polyacrylate (Flocare DP/GB 300 von SNF) | 40 |

### Beispiel 5 De-Make-up oder Reiniger

Der innere Behälter wird mit zwei Phasen teilchenförmiger Materialien gefüllt. Es wird wie im Beispiel 1 gearbeitet.

| **Phase A** | |
|---|---|
| Sodium Lauroyl Acylglutamate | 29 |
| Sorbitol | 20 |
| Allantoin | 1 |

| **Phase B** | |
|---|---|
| Sodium Polyacrylate | |
| (Flocare DP/GB 300 von SNF) | 50 |

### Beispiel 6 Duschreiniger

Es wird wie im Beispiel 1 gearbeitet.

| **Phase A** | |
|---|---|
| Tegobetaine CKD | 20 |
| Texapon K12 | 42 |

| **Phase B** | |
|---|---|
| Sodium polyacrylate (Flocare DP/GB 300 von SNF) | 38 |

### Beispiel 7 Erfrischungsmittel mit Panthenol

Die Phase A wird in einen Mischer gegeben bis maximal 70% der Mischervolumens. Die flüssige Phase B wird bei laufendem Mischer über einen Zeitraum von 10-20 Minuten horizontal oder vertikal eingesprüht. Man erhält ein trockenes Pulver, das wie im Beispiel 1 zusammen mit der Phase C in den inneren Behälter gegeben wird.

| **Phase A** | |
|---|---|
| Modifiziertes Siliciumdioxid (Sipernat® 2200) | 5 |

| **Phase B** | |
|---|---|
| Panthenol-D | 0,1 |
| Laureth-3 | 1,9 |
| Parfüm | 3 |

| **Phase C** | |
|---|---|
| Aquasorb A500 | 90 |

### Beispiel 8 Parfüm

| **Phase A** | |
|---|---|
| Modified Silica (Sipernat® 2200 | 15 |

| **Phase B** | |
|---|---|
| Parfüm | 10 |
| Laureth-3 | 5 |

| **Phase C** | |
|---|---|
| Sodium Polyacrylate (Flocare DP/GB 300 von SNF) | 70 |

### Beispiel 9 Reiniger mit Pflanzenextrakten

| **Phase A** | |
|---|---|
| Modifiziertes Siliciumdioxid (Sipernat® 2200) | 35 |

| **Phase B** | |
|---|---|
| Parfüm | 5 |
| Laureth-3 | 10 |
| Pflanzenextrakt | 20 |

| **Phase C** | |
|---|---|
| Sodium Polyacrylate (Flocare DP/GB 300 von SNF) | 30 |

### Beispiel 10 Bodylotion-Emulsion

| **Phase A** | |
|---|---|
| Sipernat 2200 | 35 |

| **Phase B** | |
|---|---|
| Bodylotion-Emulsion mit niedriger Viskosität (RVTDVII-M4 V10, 20 ° 9000-10000 Pa·s) basierend auf Pemulen TR1 | 35 |
| Sodium Polyacrylate (Flocare DP/GB 300 von SNF) | 30 |

## Patentansprüche

1. Packungseinheit mit kosmetisch wirksamen Bestandteilen, bestehend aus zwei ineinander angeordneten Behältern, wobei der erste Behälter aus einem wasserdichten Material besteht und der zweite Behälter aus einem wasserdurchlässigen Material besteht und in dem ersten Behälter angeordnet ist, und beide Behälter verschlossen sind,
**dadurch gekennzeichnet, dass** der erste Behälter standfähig ist oder zu einer standfähigen Form entfaltbar ist und Mittel zu seiner Öffnung aufweist,
und der zweite Behälter in seinem Inneren einen geträgerten oder ungeträgerten, teilchenförmigen, kosmetisch wirksamen Stoff oder ein Stoffgemisch enthält und einen zweiten teilchenförmigen Stoff enthält, der bei Kontakt mit Wasser ein wenigstens um das Vierfache vergrößertes Volumen hat.

2. Packungseinheit nach Anspruch 1, **dadurch gekennzeichnet, daß** der zweite teilchenförmige Stoff bei Kontakt mit Wasser ein wenigstens um das 10-fache vergrößertes Volumen hat.

3. Packungseinheit nach Anspruch 1, **dadurch gekennzeichnet, daß** der zweite teilchenförmige Stoff eine Absorptionsfähigkeit für Wasser im Bereich des 20- bis 50-fachen seines Trockengewichtes hat.

4. Packungseinheit nach Anspruch 1, **dadurch gekennzeichnet, daß** die Absorptionsfähigkeit für Wasser einer vorgegebenen Menge des zweiten teilchenförmigen Stoffes in einem Zeitraum von 30 bis 150 Sekunden liegt.

5. Packungseinheit nach Anspruch 1, **dadurch gekennzeichnet, daß** der zweite teilchenförmige Stoff Natriumpolyacrylat oder modifizierte Carboxymethylcellulose ist.

6. Packungseinheit nach Anspruch 1, **dadurch gekennzeichnet, daß** der erste Behälter aus einer mit PE und/oder PET beschichteten Aluminiumfolie besteht.

7. Packungseinheit nach Anspruch 1, **dadurch gekennzeichnet, daß** der zweite Behälter aus einem Faservlies mit einer Abdichtungsfähigkeit entsprechend seiner Zusammensetzung besteht.

8. Packungseinheit nach Anspruch 1, **dadurch gekennzeichnet, daß** der zweite Behälter aus Polypropylen besteht.

9. Packungseinheit nach Anspruch 1, **dadurch gekennzeichnet, daß** die kosmetisch wirksamen Substanzen, die bei Umgebungstemperatur fest sind, ausgewählt sind aus der Gruppe, bestehend aus Ascorbinsäure, Ascorbate, Dihydroxyaceton, Allantoin, sprühgetrocknete Pflanzenextrakte, verkapselte Parfümöle, verkapselte Vitamine, α-Hydroxysäuren, Glycerin, Sorbitol, Laurylethersulfat, Acyl Cocoyl Glutamate, Cocamidopropylbetaine, Deodorantien, Antiperspirantien und Gemische davon.

10. Packungseinheit nach Anspruch 1, **dadurch gekennzeichnet, daß** die kosmetischen wirksamen Substanzen, die bei Umgebungstemperatur flüssig oder halbflüssig sind, ausgewählt sind aus der Gruppe, bestehend aus Ölen, öllöslichen Substanzen, Retinol, Retinolderivaten, Tocopherol, Tocopherolderivaten, Panthenol, Pflanzenextrakten, Parfümölen, Feuchthaltemitteln (humectants), Erweichungsmitteln (emollients), Emulgatoren und Gemische davon.

11. Packungseinheit nach Anspruch 1, **dadurch gekennzeichnet, daß** der Anteil der kosmetisch annehmbaren Substanzen, die bei Umgebungstemperatur fest oder flüssig sind, im Bereich von 0,1 bis 95 Gew-% liegt, bezogen auf das Gesamtgemisch der Behälterinhalte.

12. Packungseinheit nach Anspruch 5, **dadurch gekennzeichnet, daß** die Menge an Natriumpolyacrylat oder Carboxymethylcellulose im Bereich von 10-90 Gew-% liegt, vorzugsweise 10 bis 50 Gew-%.

13. Packungseinheit nach Anspruch 1, **dadurch gekennzeichnet, daß** der kosmetisch wirksame teilchenförmige Stoff auf einem pulverförmigen Träger aufgebracht ist.

14. Packungseinheit nach Anspruch 1, **dadurch gekennzeichnet, daß** der pulverförmige Träger Siliciumdioxid oder Nylon-12 ist.

15. Verwendung der Packungseinheit nach Anspruch 1 zur Anwendung auf der Haut oder dem Haar zur Erzeugung eines Frische-Effektes.

## Claims

1. Packing unit with cosmetically active ingredients wherein two containers are arranged within each other and
where the first container consists of a water-proof material and the second container consists of a water-transmitting material and is arranged in the first container, and both containers are closed, which comprises that the first container is able to stand or can be unfolded to make it stand and includes means for opening the closed container;
and the second container has in its interior a particle-shaped, cosmetically active substance provided on a carrier or not provided on a carrier or a mixture of substances and a second particle-shaped substance with a volume that increases at least fourfold when getting into contact with water.

2. Packing unit according to claim 1 wherein the second particle-shaped substance increases its volume at least tenfold when getting into contact with water.

3. Packing unit according to claim 1 wherein the second particle-shaped substance has an absorption capacity for water within the range of the 20-fold to 50-fold of its dry weight.

4. Packing unit according to claim 1 wherein the absorption capacity for water of a pre-determined quantity of the second particle-shaped substance takes effect within a period of 30 to 150 seconds.

5. Packing unit according to claim 1 wherein the second particle-shaped substance is sodium polyacrylate or modified carboxymethylcellulose.

6. Packing unit according to claim 1 wherein the first container consists of an aluminium foil coated with PE and/or PET.

7. Packing unit according to claim 1 wherein the second container consists of a non-woven with a sealing ability due to its composition.

8. Packing unit according to claim 8 wherein the second container consists of polypropylene.

9. Packing unit according to claim 1 wherein the cosmetically active ingredients which are in a solid state at ambient temperature are selected from the group consisting of ascorbic acid, ascorbates, dihydroxy acetone, allantoin, spray-dried plant extracts, encapsulated perfume oils, encapsulated vitamins, α-hydroxy acids, glycerin, sorbitol, lauryl ether sulfate, Acyl Cocoyl Glutamate, Cocamidopropylbetaine, deodorants, antiper-spirants and mixtures thereof.

10. Packing unit according to claim 1 wherein the cosmetically active substances which are in a liquid or semi-liquid form at ambient temperature are selected from the group consisting of oils, oil-soluble substances, retinol, retinol derivatives, tocopherol, tocopherol derivatives, Panthenol, plant extracts, perfume oils, moisturisers, emollients, emulsifiers and mixtures thereof.

11. Packing unit according to claim 1 wherein the percentage of the cosmetically acceptable substances which are solid or liquid at ambient temperature is in the range of 0.1 to 95 percent by weight, calculated in relation to the total mixture of the container contents.

12. Packing unit according to claim 5 wherein the percentage of sodium polyacrylate or carboxymethylcellulose is in the range of 10 to 90 percent by weight, preferably 10 to 50 percent by weight.

13. Packing unit according to claim 1 wherein the cosmetically active particle-shaped substance is placed on a powdery carrier.

14. Packing unit according to claim 1 wherein the powdery carrier is silica or nylon-12.

15. Use of the packing unit according to claim 1 to the skin or hair for generation a fresh effect.

## Revendications

1. Unité d'emballage avec des composants cosmétiquement efficaces, constituée de deux récipients disposés l'un dans l'autre, le premier récipient étant constitué d'un matériau étanche à l'eau et le deuxième matériau étant constitué d'un matériau perméable à l'eau et étant disposé dans le premier récipient, les deux récipients étant fermés,
**caractérisée en ce que** le premier récipient peut tenir debout ou peut être transformé en une forme tenant debout et présente un dispositif pour son ouverture,
et le deuxième récipient comprend dans son espace intérieur une substance cosmétiquement active, particulaire, contenant un véhicule ou non, ou un mélange de substances, et comprend une deuxième substance particulaire qui, au contact de l'eau, a un volume augmenté au moins du quadruple.

2. Unité d'emballage selon la revendication 1, **caractérisée en ce que** la deuxième substance particulaire a, au contact de l'eau, un volume augmenté au moins du décuple.

3. Unité d'emballage selon la revendication 1, **caractérisée en ce que** la deuxième substance particulaire a une capacité d'absorption de l'eau dans la plage allant de 20 à 50 fois son poids sec.

4. Unité d'emballage selon la revendication 1, **caractérisée en ce que** la capacité d'absorption d'eau d'une quantité prédéterminée de la deuxième substance particulaire se situe sur une durée de 30 à 150 secondes.

5. Unité d'emballage selon la revendication 1, **caractérisée en ce que** la deuxième substance particulaire est le polyacrylate de sodium ou la carboxyméthylcellulose modifiée.

6. Unité d'emballage selon la revendication 1, **caractérisée en ce que** le premier récipient se compose d'une feuille d'aluminium revêtue de PE et/ou de PET.

7. Unité d'emballage selon la revendication 1, **caractérisée en ce que** le deuxième récipient se compose d'un voile non tissé de fibres ayant une capacité d'absorption correspondant à sa composition.

8. Unité d'emballage selon la revendication 1, **caractérisée en ce que** le deuxième récipient se compose de polypropylène.

9. Unité d'emballage selon la revendication 1, **caractérisée en ce que** les substances cosmétiquement actives qui sont solides à température ambiante, sont choisies dans le groupe constitué par l'acide ascorbique, les ascorbates, la dihydroxyacétone, l'allantoïne, les extraits végétaux séchés par pulvérisation, les huiles essentielles de parfum encapsulées, les vitamines encapsulées, les a-hydroxyacides, la glycérine, le sorbitol, le lauryl éther sulfate, l'acyl cocoyl glutamate, la cocamidopropylbétaïne, les déodorants, les antitranspirants et les mélanges de ceux-ci.

10. Unité d'emballage selon la revendication 1, **caractérisée en ce que** les substances cosmétiquement actives qui sont liquides ou semi-liquides à température ambiante sont choisies dans le groupe constitué par les huiles, les substances liposolubles, le rétinol, les dérivés de rétinol, le tocophérol, les dérivés de tocophérol, le panthénol, les extraits végétaux, les huiles essentielles de parfum, les agents hydratants, les émollients, les émulsifiants et les mélanges de ceux-ci.

11. Unité d'emballage selon la revendication 1, **caractérisée en ce que** la proportion de substances cosmétiquement acceptables qui sont solides ou liquides à température ambiante se situe dans la plage allant de 0,1 à 95 % en poids, par rapport au poids total des contenus des récipients.

12. Unité d'emballage selon la revendication 1, **caractérisée en ce que** la quantité de polyacrylate de sodium ou de carboxyméthylcellulose se situe dans la plage allant de 10 à 90 % en poids, de préférence 10 à 50 % en poids.

13. Unité d'emballage selon la revendication 1, **caractérisée en ce que** la substance particulaire cosmétiquement active est déposée sur un support pulvérulent.

14. Unité d'emballage selon la revendication 1, **caractérisée en ce que** le support pulvérulent est le dioxyde de silicium ou le Nylon-12.

15. Utilisation de l'unité d'emballage selon la revendication 1 pour l'utilisation sur la peau ou sur les cheveux pour produire un effet fraîcheur.
